# EUROPEAN PATENT SPECIFICATION

(11) **EP 4 017 840 B1**
(45) Date of publication and mention of the grant of the patent: **25.09.2024**
(21) Application number: 20764258.8
(22) Date of filing: 17.08.2020
(51) Int. Cl.: C07C 227/18, C08G 69/26, C07C 229/24

(54) **FAST PREPARATION OF LOW PRIMARY AMINE CONTAINING POLYASPARTIC ESTERS AND USE OF THESE POLYASPARTIC ESTERS IN SLOW REACTIVITY POLYUREA SYSTEMS**
SCHNELLE HERSTELLUNG VON SCHWACHEM PRIMÄREN AMIN MIT POLYASPARAGINSÄUREESTERN UND VERWENDUNG DIESER POLYASPARAGINSÄUREESTER IN POLYHARNSTOFFSYSTEMEN MIT LANGSAMER REAKTIVITÄT
PRÉPARATION RAPIDE D'ESTERS POLYASPARTIQUES À FAIBLE TENEUR EN AMINE PRIMAIRE ET UTILISATION DE CES ESTERS POLYASPARTIQUES DANS DES SYSTÈMES DE POLYURÉE À RÉACTIVITÉ LENTE

(30) Priority: 20.08.2019 US 201916545430
(43) Date of publication of application: 29.06.2022
(73) Proprietor: Covestro LLC, Pittsburgh, PA 15205 (US)
(72) Inventor: JOHNSTON, Jay A., Clinton, Pennsylvania 15026 (US); SQUILLER, Edward P., Bridgeville, Pennsylvania 15017 (US); WILLIAMS, Charles Todd, Lakewood, Colorado 80226 (US); STEWART, Matthew, Pittsburgh, Pennsylvania 15237 (US); GUSTAVICH, Wendy, Maynard, Ohio 43937 (US); MORRIS, Mark, Pittsburgh, Pennsylvania 15209 (US); MILLER, Adam, Pittsburgh, Pennsylvania 15214 (US)
(74) Representative: Levpat
(86) International application number: PCT/US2020/046609
(87) International publication number: WO 2021/034742

(56) References cited:
- WO-A1-01/07399
- WO-A1-2015/130502
- US-A1- 2005 075 477

## Description

### FIELD OF THE INVENTION

The present invention relates, in general, to coatings and more specifically, to fast preparation of low primary amine (LPA)-containing polyaspartic esters and use of those polyaspartic esters in slow reactivity polyurea systems.

### BACKGROUND OF THE INVENTION

Polyurethane-based or polyurea-based two-component coating systems are known to those skilled in the art. In general, such systems include a liquid polyisocyanate component and a liquid, isocyanate-reactive component. Reaction of polyisocyanates with an amine as the isocyanate-reactive component produces highly crosslinked polyurea coatings. Primary amines and isocyanate moieties, however, usually react very rapidly with one another, with typical pot lives or gelling times often being from only several seconds to a few minutes. Primary amines present in standard polyaspartic amines reacting quickly with polyisocyanates create heat and accelerate the reaction which can shorten the pot life of the resin and increase its viscosity. The fast reactivity problems of polyaspartic amines may be further exacerbated by conducting the reaction in high humidity and high temperature environments. Thus, the working time is decreased, which can cause application issues for contractors applying the coating in higher humidity/higher temperature conditions.

Various approaches have been tried to alleviate these problems. For example, Karl, et al., in WO 2018/160932, provide methods of preparing a polyaspartic ester composition comprising reacting a primary diamine reactant composition with a diester reactant composition under conditions to prepare a polyaspartic ester composition having a primary amine value of less than 35 mg KOH/g wherein, at the time of the reaction, the combined water content of the primary diamine reactant composition and the diester reactant composition is less than 300 ppm. At paragraph [0022], Karl et al., state that the combined water content of the primary diamine reactant composition and the diester reactant composition is less than 200 ppm, or less than 100 ppm, or less than 75 ppm, or less than 50 ppm, or less than 25 ppm. WO2015/130502 also discloses a coating composition and a method of coating comprising the use of mono- and diaspartic acid esters.

Therefore, a need exists in the art for a fast preparation method of low primary amine containing polyaspartic esters for use in slow reactivity polyurea systems.

### SUMMARY OF THE INVENTION

Accordingly, the present invention reduces or eliminates problems inherent in the art by providing a fast preparation method of low primary amine containing polyaspartic esters having a higher water content than those in the art. Such low primary amine containing polyaspartic esters may find use in slow reactivity polyurea systems.

These and other advantages and benefits of the present invention will be apparent from the Detailed Description of the Invention herein below.

### BRIEF DESCRIPTION OF THE FIGURES

The present invention will now be described for purposes of illustration and not limitation in conjunction with the figures, wherein:
FIG. 1 illustrates the change in the percentages of monoaspartate (monoaspartic ester amine), diaspartate (diaspartic ester amine), high molecular weight amide and diaspartate - EtOH ("diaspartate minus ethanol") in the reaction product of DIAMINE A and a 50% excess of diethyl maleate (DEM) over time measured in weeks;
FIG. 2 shows the change in the percentages of monoaspartate, diaspartate, high molecular weight amide and diaspartate - EtOH in the reaction product of DIAMINE B and a 50% excess of DEM over time measured in weeks;
FIG. 3 depicts the change in the percentages of monoaspartate, diaspartate, high molecular weight amide and diaspartate - EtOH in the reaction product of DIAMINE A and an equal amount of ethanol and a 50% excess of DEM over eight weeks;
FIG. 4 illustrates the change in the percentages of monoaspartate, diaspartate, high molecular weight amide and diaspartate - EtOH in the reaction product of DIAMINE B and an equal amount of ethanol and a 50% excess of DEM over eight weeks;
FIG. 5 depicts percentages of diamine A, high molecular weight amide, diaspartate - EtOH, monoaspartate and diaspartate over seven days after the synthesis of LPA ASPARTATE A with ethanol and excess DEM;
FIG. 6 demonstrates that the thin film evaporated LPA ASPARTATE A retains a low monoaspartate percentage after distilling off the ethanol and DEM;
FIG. 7 illustrates an exotherm comparison of ASPARTATE A and LPA ASPARTATE A made according to the invention;
FIG. 8 shows an enlarged view of a portion of the exotherm comparison of FIG. 7 over the first 10 minutes of the reaction;
FIG. 9 illustrates the viscosity change over time of a 50/50 blend of LPA ASPARTATE A and LPA ASPARTATE B reacted with polyisocyanate A versus Control (a 50/50 blend of ASPARTATE A and ASPARTATE B) reacted with polyisocyanate A;
FIG. 10 shows the exotherm of a 50/50 blend of LPA ASPARTATE A and LPA ASPARTATE B reacted with polyisocyanate A versus Control (a 50/50 blend of ASPARTATE A and ASPARTATE B) reacted with polyisocyanate A;
FIG. 11 shows the diaspartate content of ASPARTATE A variants aged at room temperature over eleven days;
FIG. 12 shows the monoaspartate content of ASPARTATE A variants aged at room temperature over eleven days;
FIG. 13 depicts the diaspartate - EtOH content of ASPARTATE A variants aged at room temperature over eleven days;
FIG. 14 illustrates the high molecular weight amide content of ASPARTATE A variants aged at room temperature over eleven days;
FIG. 15 depicts the rate of formation of diaspartate of ASPARTATE A variants; and
FIG. 16 illustrates the rate of decrease in the monoaspartate content.

### DETAILED DESCRIPTION OF THE INVENTION

The present invention will now be described for purposes of illustration and not limitation. Except in the operating examples, or where otherwise indicated, all numbers expressing quantities, percentages, and so forth in the specification are to be understood as being modified in all instances by the term "about."

Any numerical range recited in this specification is intended to include all sub-ranges of the same numerical precision subsumed within the recited range. For example, a range of "1.0 to 10.0" is intended to include all sub-ranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited in this specification is intended to include all lower numerical limitations subsumed therein and any minimum numerical limitation recited in this specification is intended to include all higher numerical limitations subsumed therein.

The grammatical articles "a", "an", and "the", as used herein, are intended to include "at least one" or "one or more", unless otherwise indicated, even if "at least one" or "one or more" is expressly used in certain instances. Thus, these articles are used in this specification to refer to one or more than one (i.e., to "at least one") of the grammatical objects of the article. By way of example, and without limitation, "a component" means one or more components, and thus, possibly, more than one component is contemplated and may be employed or used in an implementation of the described embodiments. Further, the use of a singular noun includes the plural, and the use of a plural noun includes the singular, unless the context of the usage requires otherwise.

The invention is directed to a process of producing a low primary amine (LPA) polyaspartic ester comprising reacting an aliphatic diamine with an excess of a Michael addition receptor in the presence of a C₁-C₁₀ alcohol, wherein the low primary amine (LPA) polyaspartic ester has a monoaspartate content of less than 10% and wherein the Michael addition receptor is selected from the group consisting of dimethyl maleate, diethyl maleate, dibutyl maleate, dimethyl fumarate, diethyl fumarate, dibutyl fumarate and combinations thereof.

In yet another aspect, the invention is directed to coatings, adhesives, sealants, films, composites, castings, and paints comprising the low primary amine (LPA) polyaspartic ester according to the invention.

In still another aspect, the invention is directed to a polyurea comprising a reaction product of a polyisocyanate with the low primary amine (LPA) polyaspartic ester according to the invention.

As used herein, the terms "coating composition" and "coating" refer to a mixture of chemical components that, optionally cures and, forms a coating when applied to a substrate.

The terms "adhesive" and "adhesive compound", refer to any substance that can adhere or bond two items together. Implicit in the definition of an "adhesive composition" and an "adhesive formulation" is the concept that the composition or formulation is a combination or mixture of more than one species, component or compound, which can include adhesive monomers, oligomers, and polymers along with other materials.

A "sealant composition" and a "sealant" refer to a composition which may be applied to one or more surfaces to form a protective barrier, for example, to prevent ingress or egress of solid, liquid or gaseous material or alternatively to allow selective permeability through the barrier to gas and liquid. In particular, it may provide a seal between surfaces.

A "casting composition" and a "casting" refer to a mixture of liquid chemical components which is usually poured into a mold containing a hollow cavity of the desired shape, and then allowed to solidify.

A "composite" or "composite composition" refers to a material made from one or more polymers, containing at least one other type of material (e.g., a fiber) which retains its identity while contributing desirable properties to the composite. A composite has different properties from those of the individual polymers/materials which make it up.

As used herein, the term "paint" refers to a substance used for decorating or protecting a surface, and is typically a mixture containing a solid pigment suspended in a liquid, that when applied to a surface dries to form a hard, protective coating.

"Cured," "cured composition" or "cured compound" refers to components and mixtures obtained from reactive curable original compound(s) or mixture(s) thereof which have undergone a chemical and/or physical changes such that the original compound(s) or mixture(s) is(are) transformed into a solid, substantially non-flowing material. A typical curing process may involve crosslinking.

The term "curable" means that an original compound(s) or composition material(s) can be transformed into a solid, substantially non-flowing material by means of chemical reaction, crosslinking, radiation crosslinking, or the like. Thus, compositions of the invention are curable, but unless otherwise specified, the original compound(s) or composition material(s) is(are) not cured.

As used herein, "polymer" encompasses prepolymers, oligomers and both homopolymers and copolymers; the prefix "poly" in this context referring to two or more.

As used herein, "molecular weight", when used in reference to a polymer, refers to the number average molecular weight ("Mn"), unless otherwise specified.

As used herein, the Mₙ of a polymer containing functional groups, such as a polyol, can be calculated from the functional group number, such as hydroxyl number, which is determined by end-group analysis.

As used herein, the term "aliphatic" refers to organic compounds characterized by substituted or un-substituted straight, branched, and/or cyclic chain arrangements of constituent carbon atoms. Aliphatic compounds do not contain aromatic rings as part of the molecular structure thereof.

As used herein, the term "cycloaliphatic" refers to organic compounds characterized by arrangement of carbon atoms in closed ring structures. Cycloaliphatic compounds do not contain aromatic rings as part of the molecular structure thereof. Therefore, cycloaliphatic compounds are a subset of aliphatic compounds. Therefore, the term "aliphatic" encompasses aliphatic compounds and cycloaliphatic compounds.

As used herein, "diisocyanate" refers to a compound containing two isocyanate groups. As used herein, "polyisocyanate" refers to a compound containing two or more isocyanate groups. Hence, diisocyanates are a subset of polyisocyanates.

As those skilled in the art are aware, a polyaspartic ester may be produced by reacting a straight or branched alkyl or cycloalkyl residue of a polyamine with a Michael addition receptor, i.e., an electron withdrawing group such as cyano, keto or ester (an electrophile) in a Michael addition reaction. The low primary amine (LPA) polyaspartic esters of the invention comprise the reaction product of an aliphatic diamine and at least a 50% excess of a Michael addition receptor in the presence of a C₁-C₁₀ alcohol. Preferred alcohols are alkyl alcohols such as methanol, ethanol and propanol, with ethanol being particularly preferred.

The Michael addition receptors are selected from the group consisting of dimethyl maleate, diethyl maleate, dibutyl maleate, dimethyl fumarate, diethyl fumarate, and dibutyl fumarate.

The polyamine may be an aliphatic or a cycloaliphatic diamine. Examples of suitable cycloaliphatic diamines include, but are not limited to, isophoronediamine (5-amino-(1-aminomethyl)-1,3,3-trimethylcyclohexane); 1,3-cyclohexanebis(methylamine) (1,3-BAMC); 1,4-cyclohexanebis(methylamine) (1,4-BAMC); 4,4'-diaminodicyclohexylmethane (PACM 20); bis(4-amino-3-methylcyclohexyl)methane; 3, 3'-dimethyl-4, 4'-diaminodicyclohexyl-methane (DMDC); isomers thereof; salts thereof; complexes thereof; adducts thereof; and any mixtures thereof.

The production of the low primary amine (LPA) polyaspartic ester of the invention from the aliphatic diamine and an excess of Michael addition receptor starting materials may take place within a temperature range of 0°C to 100°C in various embodiments, between 10°C, or 20°C, or 30°C, or 50°C, or 60°C to 70°C, or 80°C, or 90°C in certain embodiments. The starting materials are used in amounts such that there is an excess of the Michael addition receptor to the aliphatic diamine. In various embodiments, this excess may be at least 10%, or at least 20%, or at least 30% or at least 40%, or at least 50%, or more.

A reaction scheme of one embodiment of the invention with the cycloaliphatic diamine, 4,4'-diaminodicyclohexylmethane (PACM 20) and the Michael addition receptor, diethyl maleate (DEM), is illustrated below.

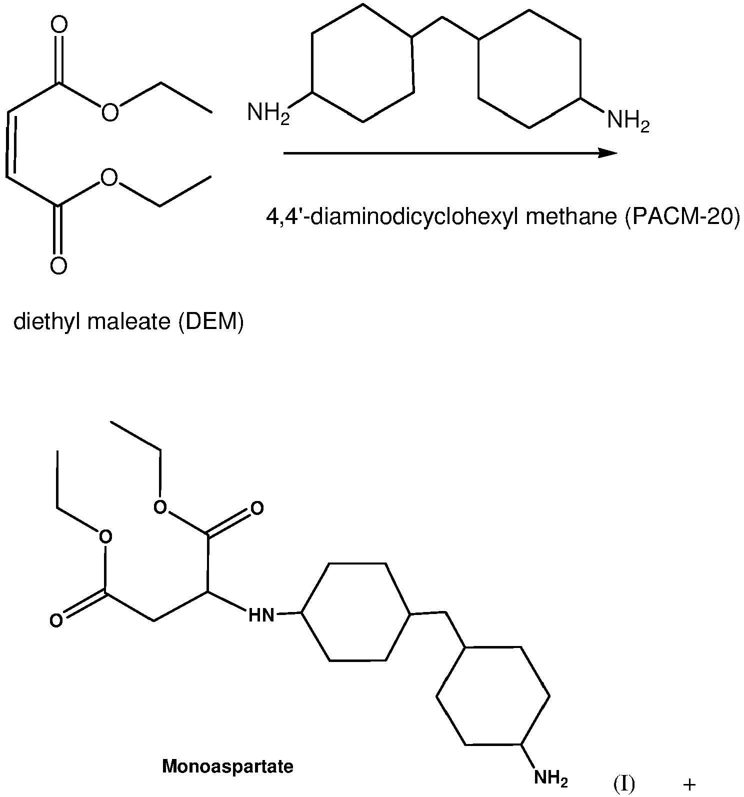

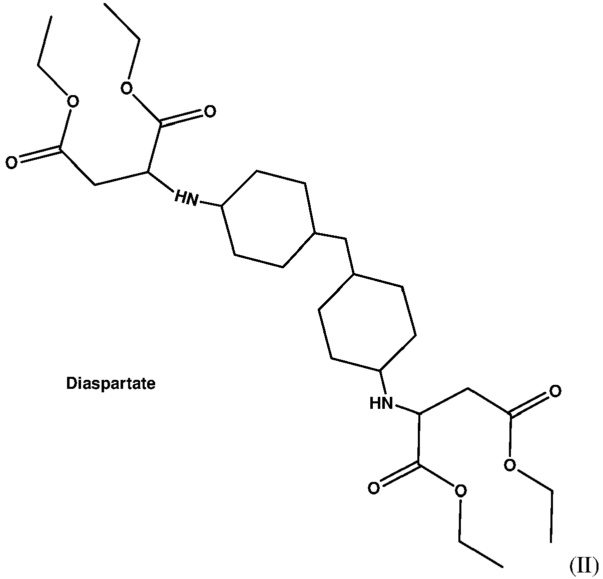

The present inventors have observed that in some instances, the monoaspartate reaction product (I) and the diaspartate reaction product (II) may combine to form a higher molecular weight amide (III), plus isomers.

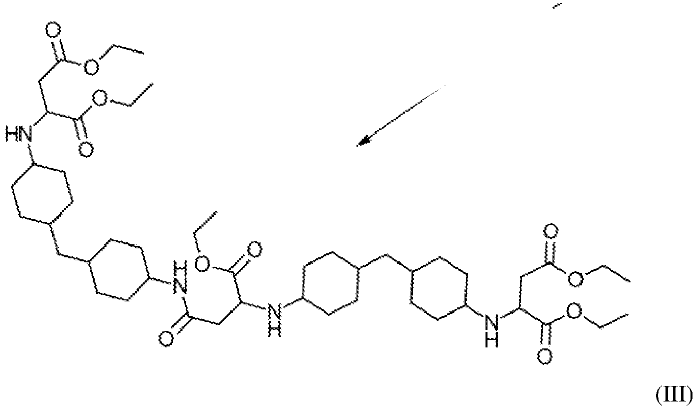

In other instances, the present inventors postulate that the diaspartate reaction product may lose an ethanol molecule and form a lower molecular weight amide, one of the proposed structures is shown as (IV). This amidization would represent an intramolecular cyclization.

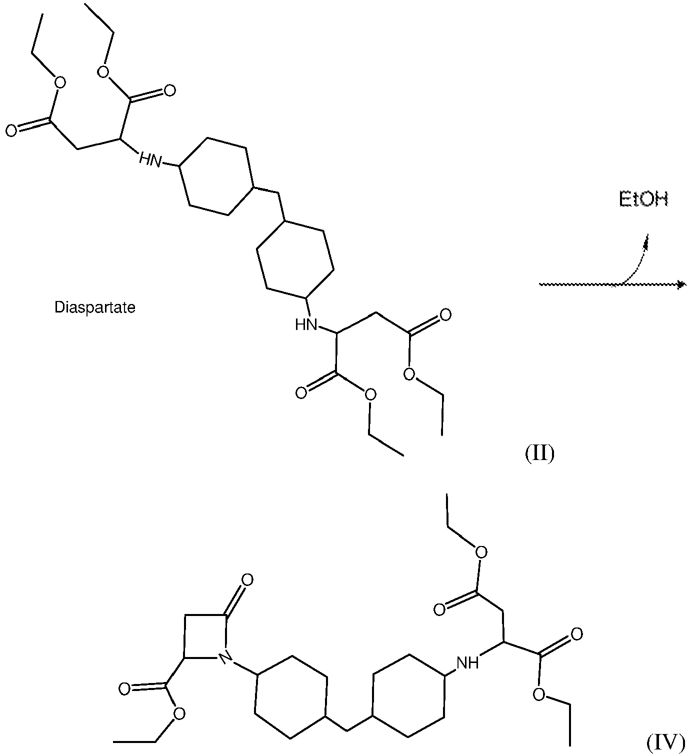

Unexpectedly, the present inventors have found that using an excess of diethyl maleate produces a low primary amine (LPA) polyaspartic ester having a lower level of monoaspartate than the conventionally produced polyaspartic esters and further that the addition of a C₁-C₁₀ alcohol (in various embodiments, methanol, ethanol, propanol) favors production of the diaspartate reaction product (II) over the monoaspartate (I). Thus, in various embodiments the inventive low primary amine (LPA) polyaspartic esters have a monoaspartate content of less than 10%, in certain embodiments less than 5% and in selected embodiments less than 3%. Polyurea coatings, adhesives, sealants, films, composites, castings, and paints may be produced from the low primary amine (LPA) polyaspartic esters according to the invention by reaction with a polyisocyanate.

Suitable polyisocyanates useful in various embodiments of the invention include organic diisocyanates represented by the formula

R(NCO)₂

wherein R represents an organic group obtained by removing the isocyanate groups from an organic diisocyanate having (cyclo)aliphatically bound isocyanate groups and a molecular weight of 112 to 1,000, preferably 140 to 400. Preferred diisocyanates for the invention are those represented by the formula wherein R represents a divalent aliphatic hydrocarbon group having from 4 to 18 carbon atoms, a divalent cycloaliphatic hydrocarbon group having from 5 to 15 carbon atoms, or a divalent araliphatic hydrocarbon group having from 7 to 15 carbon atoms.

Examples of organic diisocyanates which are particularly suitable for the present invention include 1,4-tetramethylene diisocyanate, 1,6-hexamethylene diisocyanate (HDI), 2,2,4-trimethyl-1,6-hexamethylene diisocyanate, 1,12-dodecamethylene diisocyanate, cyclohexane-1,3- and 1,4-diisocyanate, 1-isocyanato-2-isocyanato-methyl cyclopentane, 1-isocyanato-3-isocyanatomethyl-3,5,5-trimethyl cyclohexane (isophorone diisocyanate or IPDI), bis-(4-isocyanatocyclohexyl)methane, 1,3- and 1,4-bis(isocyanatomethyl)-cyclohexane, bis-(4-isocyanato-3-methyl-cyclohexyl)-methane, α,α,α',α'-tetramethyl-1,3- and 1,4-xylene diisocyanate, 1-isocyanato-1-methyl-4(3)-isocyanato-methyl cyclohexane, and 2,4- and 2,6-hexahydrotoluene diisocyanate, toluene diisocyanate (TDI), diphenylmethane diisocyanate (MDI), pentane diisocyanate (PDI) - bio-based, and, isomers or combinations of any of these. Mixtures of diisocyanates may also be used. Particularly preferred diisocyanates include 1,6-hexamethylene diisocyanate, isophorone diisocyanate, and bis(4-isocyanatocyclohexyl)-methane because they are readily available and yield relatively low viscosity oligomers.

The coatings, adhesives, sealants, films, composites, castings, and paints of the present invention may further include any of a variety of additives such as defoamers, devolatilizers, thickeners, flow control additives, colorants (including pigments and dyes), surfactants, dispersants, and neutralizers as is known to those skilled in the art.

The coatings and paints of the present invention may be admixed and combined with the conventional paint-technology binders, auxiliaries and additives, selected from the group of pigments, dyes, matting agents, flow control additives, wetting additives, slip additives, pigments, including metallic effect pigments, fillers, nanoparticles, light stabilizing particles, anti-yellowing additives, thickeners, and additives for reducing the surface tension.

The low primary amine (LPA) polyaspartic ester may find use in producing polyurea coatings, adhesives, sealants, films, composites, castings, and paints.

### EXAMPLES

The non-limiting and non-exhaustive examples that follow are intended to further describe various non-limiting and non-exhaustive embodiments without restricting the scope of the embodiments described in this specification. All quantities given in "parts" and "percents" are understood to be by weight, unless otherwise indicated. Although described herein in the context of a coating, the present invention is not to be so limited. The principles of the invention are equally applicable to coatings, adhesives, sealants, films, composites, castings, and paints.

| | |
|---|---|
| DIAMINE A | 4,4'-diaminodicyclohexylmethane, commercially available as PACM 20 from Evonik Industries; |
| DIAMINE B | 3, 3'-dimethyl-4, 4'-diaminodicyclohexylmethane, commercially available as DMDC from BASF; |
| DEM | diethyl maleate commercially available from DSM; |
| EtOH | ethanol, analytical grade, anhydrous; |
| ISOCYANATE A | an aliphatic polyisocyanate resin based on hexamethylene diisocyanate, NCO content 23.5 ± 0.5%, viscosity 730 ± 100 mPa·s @ 23°C, commercially available from Covestro as DESMODUR N-3900; |
| ASPARTATE A | a 100% solids content aspartic ester functional amine, having an amine number of ∼ 201 mg KOH/g, viscosity @ 25°C of 1450 mPa•s; |
| ASPARTATE B | a 100% solids content aspartic ester functional amine, having an amine number of approx. 191 mg KOH/g, viscosity @ 25°C of 1400; |
| LPA ASPARTATE A | a proprietary low primary amine version of ASPARTATE A made according to the invention, having a water content after stripping of EtOH and DEM of 0.021% (+/- 0.02); |
| LPA ASPARTATE B | a proprietary low primary amine version of ASPARTATE B made according to the invention, having a water content after stripping of EtOH and DEM of 0.028% (+/- 0.03); |
| TBA | t-butyl acetate; and |
| ADDITIVE A | a flow promoter and de-aerator, commercially available from OMG Americas, Inc. as BORCHI GOL 0011. |

Referring to Table I, Example A; DIAMINE A was reacted with a 50% excess of DEM to produce a low primary amine (LPA) polyaspartic ester. The composition of Example A exhibited an increase in diaspartate - EtOH ("diaspartate minus ethanol").

In Example B, DIAMINE A was reacted with a 50% excess of DEM and ethanol to produce a low primary amine (LPA) polyaspartic ester. The composition of Example B exhibited no increase in high molecular weight amide.

In Example C, DIAMINE B was reacted with a 50% excess of DEM to produce a polyaspartic ester. The composition of Example C exhibited an increase in diaspartate - EtOH.

In Example D, DIAMINE B was reacted with a 50% excess of DEM and ethanol to produce a low primary amine (LPA) polyaspartic ester. The composition of Example D exhibited no increase in high molecular weight amide.

**Table I**

| | **Ex. A** | **Ex. B** | **Ex. C** | **Ex. D** |
|---|---|---|---|---|
| DIAMINE A | 227.55g | 227.55g | | |
| DIAMINE B | | | 368.01g | 368.01g |
| DEM | 558.67g | 558.67g | 797.98g | 797.98g |
| EtOH | | 227.55g | | 368.01g |

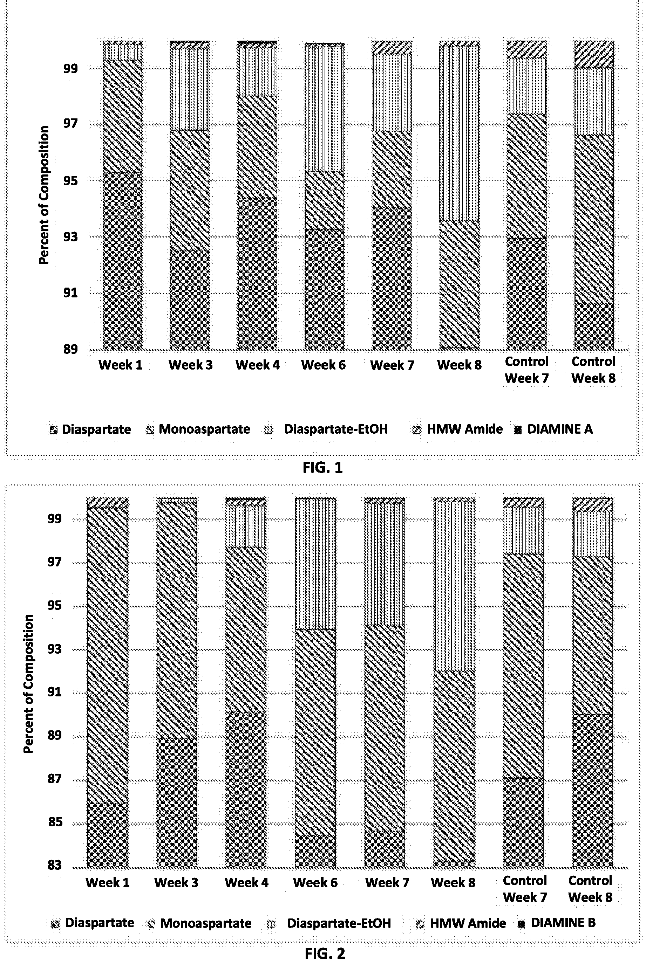

FIG. 1 illustrates the change in the percentages, as determined by LC-MS (liquid chromatography-mass spectrometry) of monoaspartate, diaspartate, and diaspartate - EtOH in the reaction product of DIAMINE A and a 50% excess of DEM over time measured in weeks (Ex. A). ASPARTATE A is included in FIG. 1 as a Control at 7 and 8 weeks. As can be appreciated by reference to FIG. 1, excess DEM yielded a reaction product having less monoaspartate but more diaspartate - EtOH.

FIG. 2 shows the change in the percentages of monoaspartate, diaspartate, and diaspartate - EtOH in the reaction product of DIAMINE B and a 50% excess of DEM over time measured in weeks (Ex. C). ASPARTATE B is included in FIG. 2 as a Control at 7 and 8 weeks. As can be appreciated by reference to FIG. 2, the resultant reaction product has more diaspartate - EtOH, undergoes a slower reaction and formation of diaspartate - EtOH, and had similar monoaspartate content compared to Control.

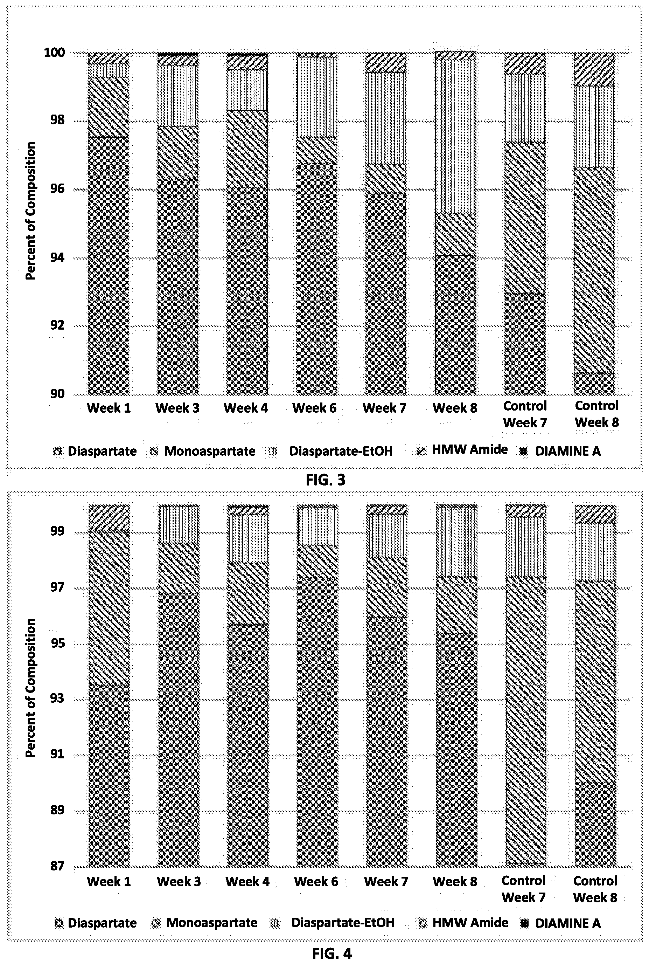

FIG. 3 depicts the change in the percentages of monoaspartate, diaspartate, and diaspartate - EtOH in the reaction product of DIAMINE A and a 50% excess DEM + EtOH over eight weeks (Ex. B). ASPARTATE A is included in FIG. 3 as a Control at 7 and 8 weeks. As can be appreciated by reference to FIG. 3, the reaction is complete in the first week, although the formation of diaspartate - EtOH continues over time, and the monoaspartate content of Ex. B was lower than the Control.

FIG. 4 illustrates the change in the percentages of monoaspartate, diaspartate and diaspartate - EtOH in the reaction product of DIAMINE B and a 50% excess of DEM + EtOH over eight weeks (Ex. D). ASPARTATE B is included in FIG. 4 as a Control at 7 and 8 weeks. As can be appreciated by reference to FIG. 4, the reaction is completed between week one and week three, with formation of diaspartate - EtOH occurring over time. The monoaspartate content was lower than the Controls.

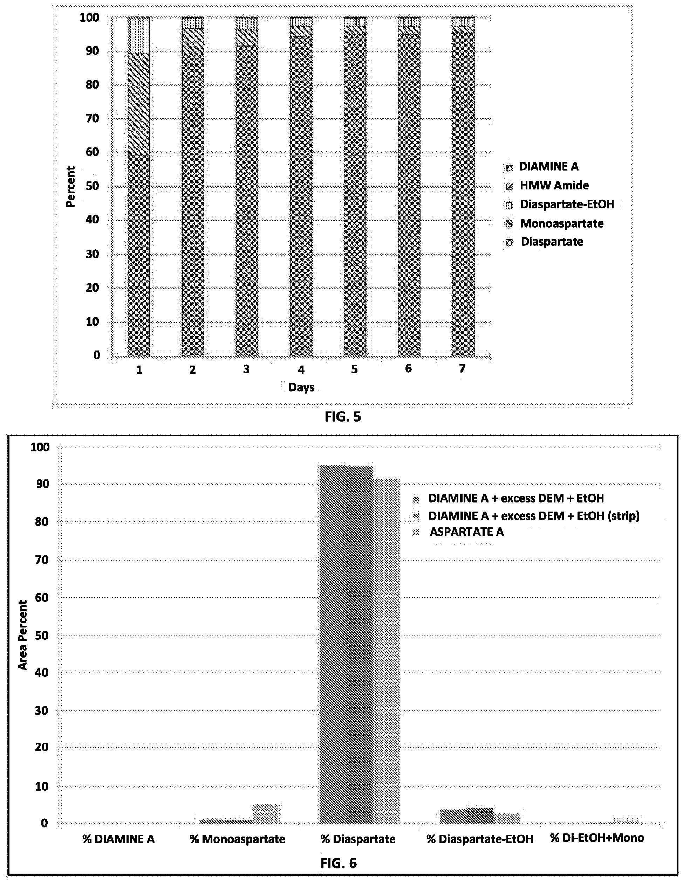

FIG. 5 depicts percentages of diamine, amide, monoaspartate, diaspartate - EtOH, and diaspartate over a seven day synthesis of LPA ASPARTATE A with EtOH and excess DEM. FIG. 6 demonstrates that the thin film evaporated LPA ASPARTATE A retained a low monoaspartate percentage after stripping away the EtOH and DEM.

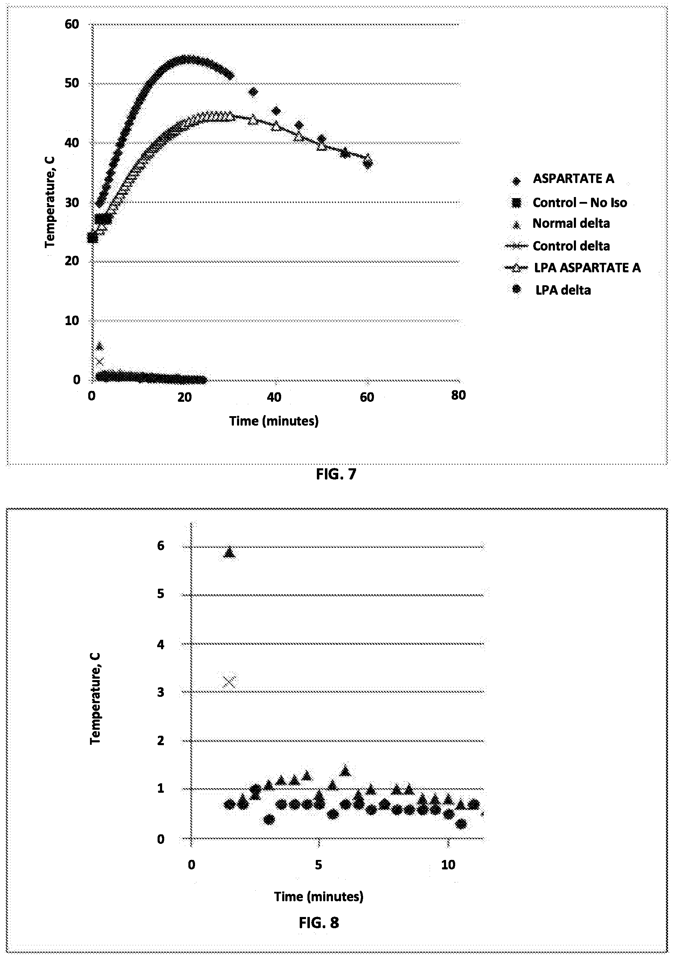

FIG. 7 illustrates an exotherm comparison of ASPARTATE A and a LPA ASPARTATE A made according to the invention. ISOCYANATE A (8.2 g) was reacted with either ASPARTATE A (11.8 g) or LPA ASPARTATE A (11.8 g) and the exotherm measured with a J-KEM Scientific Temperature Controller and a temperature probe. FIG. 8 shows an enlarged view, between 0°C and 6°C, of the exotherm comparison of FIG. 7. As can be appreciated by reference to FIG. 7 and FIG. 8, the primary amine in ASPARTATE A reacts immediately creating a spike in temperature.

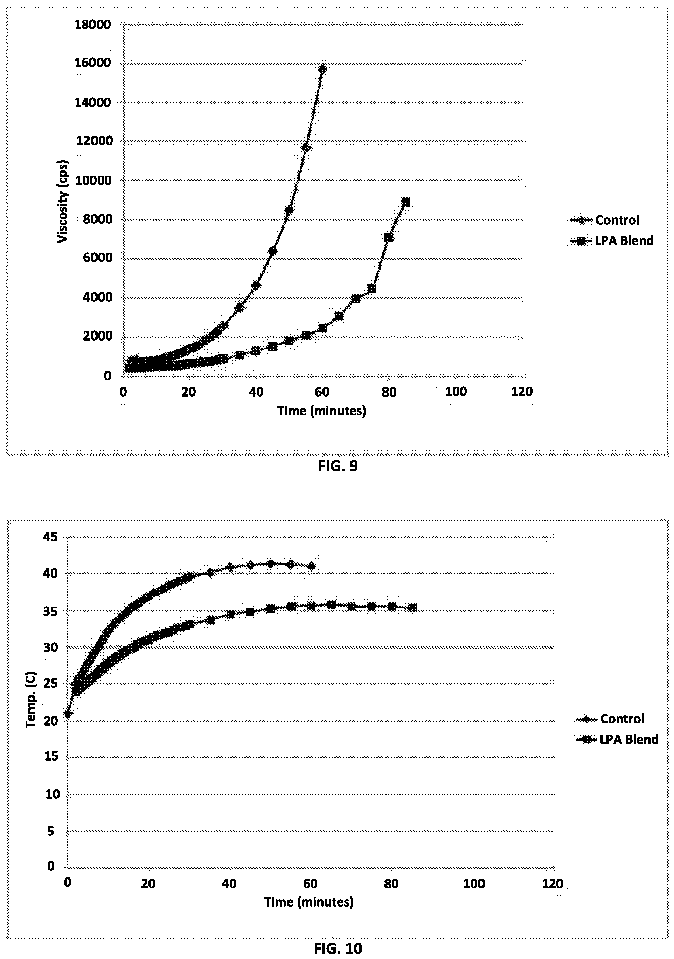

FIG. 9 illustrates the viscosity change over time of a 50/50 blend of LPA ASPARTATE A and LPA ASPARTATE B versus Control (a 50/50 blend of ASPARTATE A and ASPARTATE B) (see Table II). The viscosity was measured with a Brookfield viscometer. As can be appreciated by reference to FIG. 9, removal of the primary amine slows the molecular weight buildup as measured by viscosity.

**Table II**

| **Ingredient** | **Amount (g)** | **Amount (g)** |
|---|---|---|
| ASPARTATE A | 49.28 | |
| ASPARTATE B | 49.28 | |
| LPA A | | 49.28 |
| LPA B | | 49.28 |
| ADDITIVE A | 1.721 | 1.721 |
| TBA | 8.619 | 8.619 |
| ISOCYANATE A | 66.1 | 66.1 |

FIG. 10 shows the exotherm of a 50/50 blend of LPA ASPARTATE A and LPA ASPARTATE B versus Control (a 50/50 blend of ASPARTATE A and ASPARTATE B) (see Table II), measured by J-KEM Scientific Temperature Controller and a temperature probe. As can be appreciated by reference to FIG. 10, removal of the primary amine lowers the exotherm.

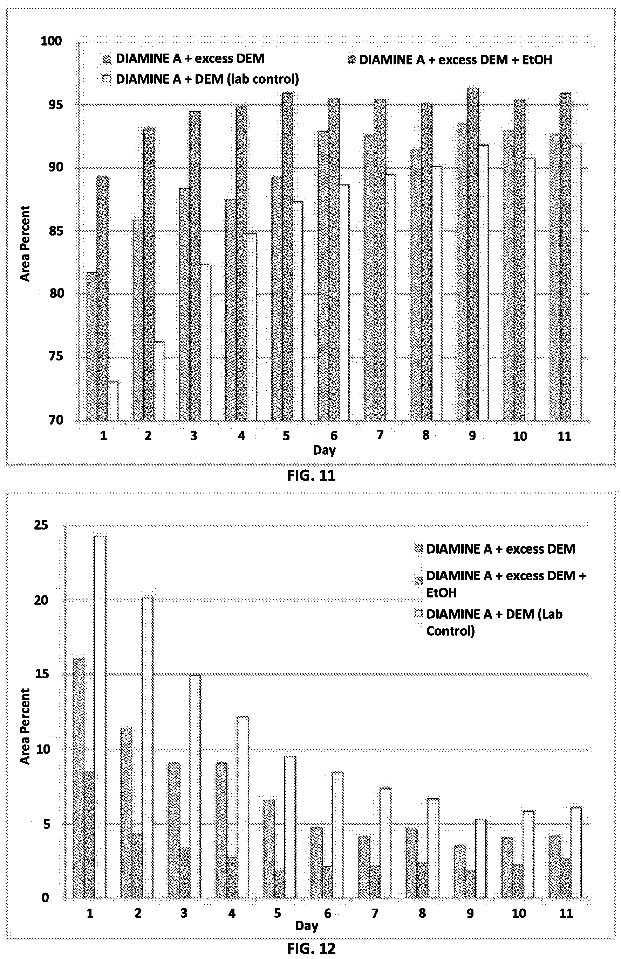

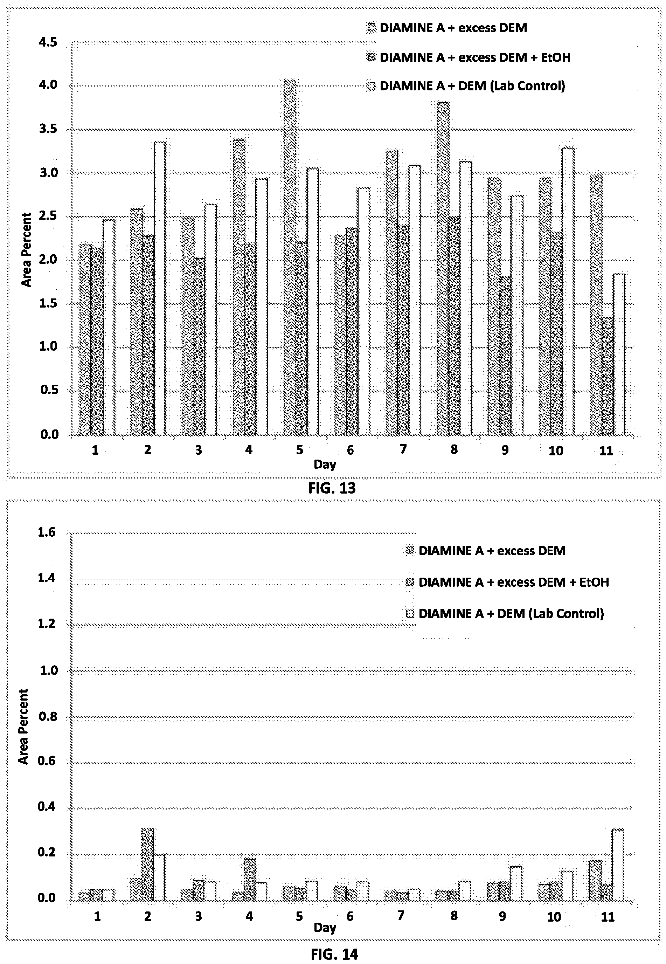

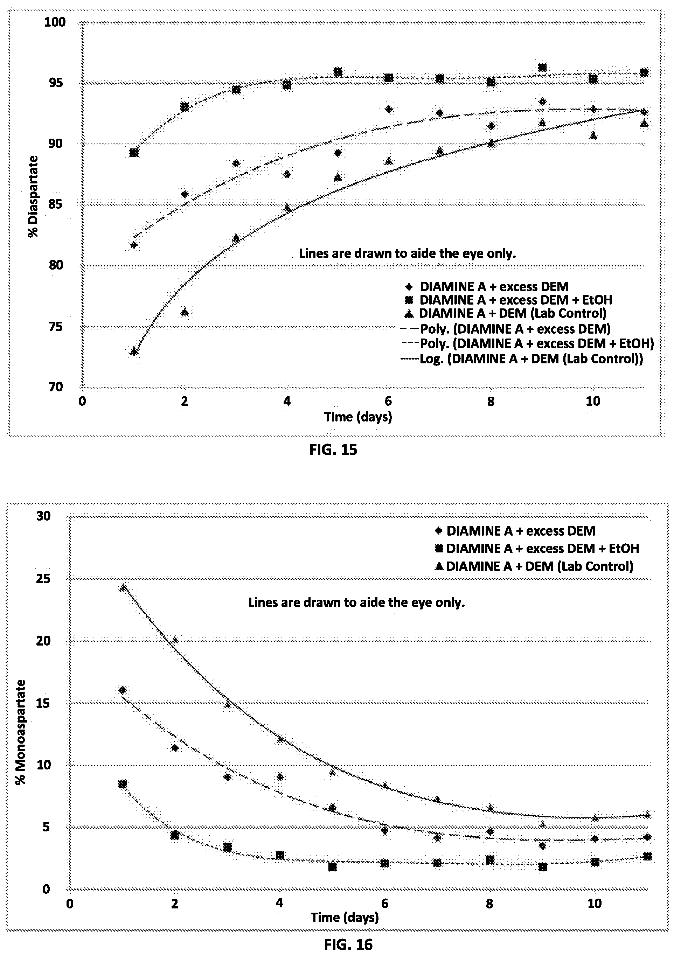

FIG. 11 illustrates the room temperature aging of aspartates- ASPARTATE A variants-diaspartate over 11 days. FIG. 12 shows the room temperature aging of aspartates-ASPARTATE A variants-monoaspartates over 11 days. FIG. 13 depicts the room temperature aging of aspartates-ASPARTATE A variants-diaspartate-EtOH over 11 days. FIG. 14 shows the room temperature aging of aspartates-ASPARTATE A variants-diaspartate-EtOH + monoaspartates over 11 days. FIG. 15 depicts diaspartate rate (% diaspartate vs. time in days) and FIG. 16 illustrates monoaspartate rate (% monoaspartate vs. time in days).

The present inventors conducted a titration to determine the amount of primary amine (in mg KOH/g) and the percentage of primary amine in each of ASPARTATE A, LPA ASPARTATE A, and DIAMINE A. As can be appreciated by reference to Table III, LPA ASPARTATE A had markedly lower percentage of primary amine compared to ASPARTATE A. DIAMINE A as expected was essentially all primary amine.

**Table III**

| **Material** | **Primary amine (mg KOH/g)** | **Primary amine (% of total amine)** |
|---|---|---|
| ASPARTATE A | 18.05 | 9.06 |
| LPA ASPARTATE A | 7.24 | 3.86 |
| DIAMINE A | 7.81 | 98.99 |

As summarized in Table IV, a 50 /50 blend of ASPARTATE A and ASPARTATE B reacted with ISOCYANATE A produced material having a gel time (determined by Gardco Standard Gel Timer, Paul N Gardner Company, Pompano Beach, FL USA) of 143:51 minutes. Adding 1000 ppm water reduced the gel time to 42:06 minutes.

**Table IV**

| | **Ex. IV-1** | **Ex. IV-2** | **Ex. IV-3** | **Ex. IV-4** |
|---|---|---|---|---|
| **Component 1** | | | | |
| ASPARTATE A | 28.33 | 28.33 | 0 | 0 |
| LPA ASPARTATE A | 0 | 0 | 28.33 | 28.33 |
| ASPARTATE B | 28.33 | 28.33 | 0 | 0 |
| LPA ASPARTATE B | 0 | 0 | 28.33 | 28.33 |
| TBA | 5 | 5 | 5 | 5 |
| water | 0 | 0.1 | 0 | 0.1 |

| **Component 2** | | | | |
|---|---|---|---|---|
| ISOCYANATE A | 38.33 | 38.33 | 38.33 | 38.33 |
| **Total** | 99.99 | 100.09 | 99.99 | 100.09 |
| | | | | |
| Gardner Gel Time (min) | 143:51 | 42:06 | 195:03 | 67:42 |
| Ratio | 3.41 | | 2.893 | |

Again, with reference to Table IV, LPA ASPARTATE A and LPA ASPARTATE B in a 50/50 blend produced material having a gel time (determined by Gardco Standard Gel Timer, Paul N Gardner Company, Pompano Beach, FL USA) of 195:03 minutes. The addition of 1000 ppm water reduced the gel time to 67:42 minutes.

As can be appreciated by reference to Table IV, reducing the percentage of primary amine in the polyaspartate increased the gel time (determined by Gardco Standard Gel Timer, Paul N Gardner Company, Pompano Beach, FL USA) and the addition of water catalyzed the reaction. The gel time ratios appeared to be similar.

Work times and walk on times for commercial polyaspartic esters and their LPA versions were assessed. Test floor coating formulations were made by combining the ingredients in the amounts listed in Table V.

**Table V**

| | **Ex. V-1** | **Ex. V-2** |
|---|---|---|
| **Component 1** | | |
| ASPARTATE A | 33.79 | 0 |
| LPA ASPARTATE A | 0 | 33.79 |
| ASPARTATE B | 33.79 | 0 |
| LPA ASPARTATE B | 0 | 33.79 |
| TBA | 5.91 | 5.91 |
| ADDITIVE A | 1.18 | 1.18 |

| **Component 2** | | |
|---|---|---|
| ISOCYANATE A | 45.32 | 45.32 |

The formulation exemplified by Example V-1 using commercially available polyaspartic esters, had a work time of approximately ten minutes. Assessing the walk-on time, at five hours a boot mark made on the surface of the coating disappeared whereas at six hours, no boot mark was made. Work time was assessed by applying the formulation to a prepped MASONITE board with a roller. A strip was applied approximately 12.7 cm (5 in.) wide and 0.203 mm (8 mils) thick. Every five minutes another 12.7 cm (5 in.) strip was applied overlapping the two coating edges. When the wet edges no longer blended together (as observed after cure), the end of the work time was reached. Or stated more succinctly, when the lap line flow back stopped and appeared as a line in the cured coating, the work time had been surpassed. Walk on time was tested by applying 0.203 mm (8 mils) of the coating onto a MASONITE board. A 91 KG (200 lb.) individual stepped onto the board at timed intervals. Each step was on an untested section of coating. Walk on time was achieved when no mark or impression was left on the coating after cure.

By comparison, the low primary amine (LPA) polyaspartic ester formulation, Example V-2, had a work time of approximately 17.5 minutes. As to walk-on time for the LPA formulation, at five hours, a slight boot mark could be made to the surface whereas at six hours no mark was made.

Thus, the work time was nearly doubled with the low primary amine (LPA) polyaspartic ester formulation while the walk-on time stayed essentially the same - offering contractors longer work times with no reduction in productivity.

This specification has been written with reference to various non-limiting and non-exhaustive embodiments. However, it will be recognized by persons having ordinary skill in the art that various substitutions, modifications, or combinations of any of the disclosed embodiments (or portions thereof) may be made within the scope of this specification. Thus, it is contemplated and understood that this specification supports additional embodiments not expressly set forth herein. Such embodiments may be obtained, for example, by combining, modifying, or reorganizing any of the disclosed steps, components, elements, features, aspects, characteristics, limitations, and the like, of the various non-limiting embodiments described in this specification. In this manner, Applicant reserves the right to amend the claims during prosecution to add features as variously described in this specification, and such amendments comply with the requirements of 35 U.S.C. §112(a), and 35 U.S.C. §132(a).

## Claims

1. A low primary amine (LPA) polyaspartic ester comprising a reaction product of an aliphatic diamine, preferably a cycloaliphatic diamine, and an excess of a Michael addition receptor, in the presence of a C₁-C₁₀ alcohol, preferably ethanol
wherein the low primary amine (LPA) polyaspartic ester has a monoaspartate content of less than 10%, preferably less than 5%, particularly preferably less than 3%,
wherein the Michael addition receptor is selected from the group consisting of dimethyl maleate, diethyl maleate, dibutyl maleate, dimethyl fumarate, diethyl fumarate, dibutyl fumarate, and combinations thereof.

2. The low primary amine (LPA) polyaspartic ester according to the preceding claim, wherein the cycloaliphatic diamine is selected from the group consisting of isophoronediamine (5-amino-(1-aminomethyl)-1,3,3-trimethylcyclohexane), 1,3-cyclohexanebis(methylamine), 1,4-cyclohexanebis(methylamine), 4,4'-diaminodicyclohexylmethane (PACM 20), bis(4-amino-3-methylcyclohexyl)methane, 3, 3'-dimethyl-4, 4'-diaminodicyclohexyl-methane (DMDC), isomers thereof, salts thereof, complexes thereof, adducts thereof, and any mixtures thereof.

3. The low primary amine (LPA) polyaspartic ester according to any of the preceding claims, wherein the Michael addition receptor is included in an excess of at least 50%.

4. One of a coating, an adhesive, a sealant, a film, a composite, a casting, and a paint comprising the low primary amine (LPA) polyaspartic ester according to any of the preceding claims.

5. A polyurea comprising the reaction product of a polyisocyanate and the low primary amine (LPA) polyaspartic ester according to any of claims 1 to 3.

6. The polyurea according to claim 5, wherein the polyisocyanate is selected from the group consisting of 1,4-tetramethylene diisocyanate, 1,6-hexamethylene diisocyanate, 2,2,4-trimethyl-1,6-hexamethylene diisocyanate, 1,12-dodecamethylene diisocyanate, cyclohexane-1,3- and 1,4-diisocyanate, 1-isocyanato-2-isocyanato-methyl cyclopentane, 1-isocyanato-3-isocyanatomethyl-3,5,5-trimethyl cyclohexane (isophorone diisocyanate), bis-(4-isocyanatocyclohexyl)methane, 1,3- and 1,4-bis(isocyanatomethyl)-cyclohexane, bis-(4-isocyanato-3-methyl-cyclohexyl)-methane, α,α,α',α'-tetramethyl-1,3- and 1,4-xylene diisocyanate, 1-isocyanato-1-methyl-4(3)-isocyanato-methyl cyclohexane, and 2,4- and 2,6-hexahydrotoluene diisocyanate, toluene diisocyanate, diphenylmethane diisocyanate, pentane diisocyanate - bio-based, and isomers or combinations of any of these.

7. A process of producing a polyurea comprising reacting a polyisocyanate with the low primary amine (LPA) polyaspartic ester according to any of claims 1 to 3.

8. The process according to claim 7, wherein the polyisocyanate is selected from the group consisting of 1,4-tetramethylene diisocyanate, 1,6-hexamethylene diisocyanate, 2,2,4-trimethyl-1,6-hexamethylene diisocyanate, 1,12-dodecamethylene diisocyanate, cyclohexane-1,3- and 1,4-diisocyanate, 1-isocyanato-2-isocyanato-methyl cyclopentane, 1-isocyanato-3-isocyanatomethyl-3,5,5-trimethyl cyclohexane (isophorone diisocyanate), bis-(4-isocyanatocyclohexyl)methane, 1,3- and 1,4-bis(isocyanatomethyl)-cyclohexane, bis-(4-isocyanato-3-methyl-cyclohexyl)-methane, α,α,α',α'-tetramethyl-1,3- and 1,4-xylene diisocyanate, 1-isocyanato-1-methyl-4(3)-isocyanato-methyl cyclohexane, and 2,4- and 2,6-hexahydrotoluene diisocyanate, toluene diisocyanate, diphenylmethane diisocyanate, pentane diisocyanate - bio-based, and isomers or combinations of any of these.

9. A process of producing a low primary amine (LPA) polyaspartic ester comprising reacting an aliphatic diamine, preferably a cycloaliphatic diamine, with an excess of a Michael addition receptor, in the presence of a C₁-C₁₀ alcohol, preferably ethanol
wherein the low primary amine (LPA) polyaspartic ester has a monoaspartate content of less than 10%, preferably less than 5%, particularly preferably less than 3%,
wherein the Michael addition receptor is selected from the group consisting of dimethyl malonate, diethyl malonate, dibutyl malonate, dimethyl fumarate, diethyl fumarate, dibutyl fumarate, and combinations thereof.

10. The process according to claim 9, wherein the cycloaliphatic diamine is selected from the group consisting of isophoronediamine (5-amino-(1-aminomethyl)-1,3,3-trimethylcyclohexane), 1,3-cyclohexanebis(methylamine), 1,4-cyclohexanebis(methylamine), 4,4'-diaminodicyclohexylmethane (PACM 20), bis(4-amino-3-methylcyclohexyl)methane, 3, 3'-dimethyl-4, 4'-diaminodicyclohexyl-methane (DMDC), isomers thereof, salts thereof, complexes thereof, adducts thereof, and any mixtures thereof.

11. The process according to any of claims 9 to 10, wherein the Michael addition receptor is included in an excess of at least 50%.

12. One of a coating, an adhesive, a sealant, a film, a composite, a casting, and a paint comprising the low primary amine (LPA) polyaspartic ester made according to the process of any of claims 9 to 11.

## Patentansprüche

1. Polyasparaginsäureester mit geringem Gehalt an primärem Amin (LPA), umfassend ein Umsetzungsprodukt eines aliphatischen Diamins, vorzugsweise eines cycloaliphatischen Diamins, und eines Überschusses eines Michael-Additions-Rezeptors in Gegenwart eines C₁-C₁₀-Alkohols, vorzugsweise Ethanol,
wobei der Polyasparaginsäureester mit geringem Gehalt an primärem Amin (LPA) einen Monoaspartatgehalt von weniger als 10 %, vorzugsweise weniger als 5 %, besonders bevorzugt weniger als 3 %, aufweist,
wobei der Michael-Additions-Rezeptor aus der Gruppe bestehend aus Dimethylmaleat, Diethylmaleat, Dibutylmaleat, Dimethylfumarat, Diethylfumarat, Dibutylfumarat und Kombinationen davon ausgewählt ist.

2. Polyasparaginsäureester mit geringem Gehalt an primärem Amin (LPA) nach dem vorhergehenden Anspruch, wobei das cycloaliphatische Diamin aus der Gruppe bestehend aus Isophorondiamin (5-Amino-(1-aminomethyl)-1,3,3-trimethylcyclohexan), 1,3-Cyclohexanbis(methylamin), 1,4-Cyclohexanbis(methylamin), 4,4'-Diaminodicyclohexylmethan (PACM 20), Bis(4-amino-3-methylcyclohexyl)methan, 3,3'-Dimethyl-4,4'-diaminodicyclohexylmethan (DMDC), Isomere davon, Salzen davon, Komplexen davon, Addukten davon und beliebigen Mischungen davon ausgewählt ist.

3. Polyasparaginsäureester mit geringem Gehalt an primärem Amin (LPA) nach einem der vorhergehenden Ansprüche, wobei der Michael-Additions-Rezeptor in einem Überschuss von mindestens 50 ö enthalten ist.

4. Beschichtung, Klebstoff, Dichtungsmittel, Film, Verbundwerkstoff, Vergussmasse oder Anstrichmittel, umfassend den Polyasparaginsäureester mit geringem Gehalt an primärem Amin (LPA) nach einem der vorhergehenden Ansprüche.

5. Polyharnstoff, umfassend das Umsetzungsprodukt eines Polyisocyanats und des Polyasparaginsäureesters mit geringem Gehalt an primärem Amin (LPA) nach einem der Ansprüche 1 bis 3.

6. Polyharnstoff nach Anspruch 5, wobei das Polyisocyanat aus der Gruppe bestehend aus 1,4-Tetramethylendiisocyanat, 1,6-Hexamethylendiisocyanat, 2,2,4-Trimethyl-1,6-hexamethylendiisocyanat, 1,12-Dodecamethylendiisocyanat, Cyclohexan-1,3- und -1,4-diisocyanat, 1-Isocyanato-2-isocyanatomethylcyclopentan, 1-Isocyanato-3-isocyanatomethyl-3,5,5-trimethylcyclohexan (Isophorondiisocyanat), Bis(4-isocyanatocyclohexyl)methan, 1,3- und 1,4-Bis(isocyanatomethyl)cyclohexan, Bis(4-isocyanato-3-methylcyclohexyl)methan, α,α,α',α'-Tetramethyl-1,3- und -1,4-xylendiisocyanat, 1-Isocyanato-1-methyl-4(3)-isocyanatomethylcyclohexan und 2,4- und 2,6-Hexahydrotoluoldiisocyanat, Toluoldiisocyanat, Diphenylmethandiisocyanat, Pentandiisocyanat - biobasiert und Isomeren oder Kombinationen von beliebigen davon ausgewählt ist.

7. Verfahren zur Herstellung eines Polyharnstoffs, umfassend das Umsetzen eines Polyisocyanats mit dem Polyasparaginsäureester mit geringem Gehalt an primärem Amin (LPA) nach einem der Ansprüche 1 bis 3.

8. Verfahren nach Anspruch 7, wobei das Polyisocyanat aus der Gruppe bestehend aus 1,4-Tetramethylendiisocyanat, 1,6-Hexamethylendiisocyanat, 2,2,4-Trimethyl-1,6-hexamethylendiisocyanat, 1,12-Dodecamethylendiisocyanat, Cyclohexan-1,3- und -1,4-diisocyanat, 1-Isocyanato-2-isocyanatomethylcyclopentan, 1-Isocyanato-3-isocyanatomethyl-3,5,5-trimethylcyclohexan (Isophorondiisocyanat), Bis(4-isocyanatocyclohexyl)methan, 1,3- und 1,4-Bis(isocyanatomethyl)cyclohexan, Bis(4-isocyanato-3-methylcyclohexyl)methan, α,α,α',α'-Tetramethyl-1,3- und -1,4-xylendiisocyanat, 1-Isocyanato-1-methyl-4(3)-isocyanatomethylcyclohexan und 2,4- und 2,6-Hexahydrotoluoldiisocyanat, Toluoldiisocyanat, Diphenylmethandiisocyanat, Pentandiisocyanatbiobasiert und Isomeren oder Kombinationen von beliebigen davon ausgewählt wird.

9. Verfahren zur Herstellung eines Polyasparaginsäureesters mit geringem Gehalt an primärem Amin (LPA), umfassend das Umsetzen eines aliphatischen Diamins, vorzugsweise eines cycloaliphatischen Diamins, mit einem Überschuss eines Michael-Additions-Rezeptors in Gegenwart eines C₁-C₁₀-Alkohols, vorzugsweise Ethanol, wobei der Polyasparaginsäureester mit geringem Gehalt an primärem Amin (LPA) einen Monoaspartatgehalt von weniger als 10 %, vorzugsweise weniger als 5 %, besonders bevorzugt weniger als 3 %, aufweist,
wobei der Michael-Additions-Rezeptor aus der Gruppe bestehend aus Dimethylmalonat, Diethylmalonat, Dibutylmalonat, Dimethylfumarat, Diethylfumarat, Dibutylfumarat und Kombinationen davon ausgewählt ist.

10. Verfahren nach Anspruch 9, wobei das cycloaliphatische Diamin aus der Gruppe bestehend aus Isophorondiamin (5-Amino-(1-aminomethyl)-1,3,3-trimethylcyclohexan), 1,3-Cyclohexanbis(methylamin), 1,4-Cyclohexanbis(methylamin), 4,4'-Diaminodicyclohexylmethan (PACM 20), Bis(4-amino-3-methylcyclohexyl)methan, 3,3'-Dimethyl-4,4'-diaminodicyclohexylmethan (DMDC), Isomeren davon, Salzen davon, Komplexen davon, Addukten davon und beliebigen Mischungen davon ausgewählt wird.

11. Verfahren nach einem der Ansprüche 9 bis 10, wobei der Michael-Additions-Rezeptor in einem Überschuss von mindestens 50 ö enthalten ist.

12. Beschichtung, Klebstoff, Dichtungsmittel, Film, Verbundwerkstoff, Vergussmasse oder Anstrichmittel, umfassend den nach dem Verfahren nach einem der Ansprüche 9 bis 11 hergestellten Polyasparaginsäureester mit geringem Gehalt an primärem Amin (LPA).

## Revendications

1. Ester polyaspartique à faible teneur en amine primaire (LPA) comprenant un produit de réaction d'une diamine aliphatique, préférablement d'une diamine cycloaliphatique, et d'un excès d'un récepteur d'addition de Michael, en la présence d'un alcool en C₁-C₁₀, préférablement l'éthanol,
l'ester polyaspartique à faible teneur en amine primaire (LPA) ayant une teneur en monoaspartate inférieure à 10 %, préférablement inférieure à 5 %, particulièrement préférablement inférieure 3 %,
le récepteur d'addition de Michael étant choisi dans le groupe constitué par le maléate de diméthyle, le maléate de diéthyle, le maléate de dibutyle, le fumarate de diméthyle, le fumarate de diéthyle, le fumarate de dibutyle et des combinaisons correspondantes.

2. Ester polyaspartique à faible teneur en amine primaire (LPA) selon la revendication précédente, la diamine cycloaliphatique étant choisie dans le groupe constitué par isophoronediamine (5-amino-(1-aminométhyl)-1,3,3-triméthylcyclohexane), 1,3-cyclohexanebis(méthylamine), 1,4-cyclohexanebis(méthylamine), 4,4'-diaminodicyclohexylméthane (PACM 20), bis(4-amino-3-méthylcyclohexyl)méthane, 3, 3'-diméthyl-4, 4'-diaminodicyclohexyl-méthane (DMDC), des isomères correspondants, des sels correspondants, des complexes correspondants, des adduits correspondants et de quelconques mélanges correspondants.

3. Ester polyaspartique à faible teneur en amine primaire (LPA) selon l'une quelconque des revendications précédentes, le récepteur d'addition de Michael étant compris en un excès d'au moins 50 %.

4. Revêtement, adhésif, agent d'étanchéité, film, composite, moulage ou peinture comprenant l'ester polyaspartique à faible teneur en amine primaire (LPA) selon l'une quelconque des revendications précédentes.

5. Polyurée comprenant le produit de réaction d'un polyisocyanate et de l'ester polyaspartique à faible teneur en amine primaire (LPA) selon l'une quelconque des revendications 1 à 3.

6. Polyurée selon la revendication 5, le polyisocyanate étant choisi dans le groupe constitué par diisocyanate de 1,4-tétraméthylène, diisocyanate de 1,6-hexaméthylène, diisocyanate de 2,2,4-triméthyl-1,6-hexaméthylène, diisocyanate de 1,12-dodécaméthylène, 1,3- et 1,4-diisocyanate de cyclohexane, 1-isocyanato-2-isocyanato-méthylcyclopentane, 1-isocyanato-3-isocyanatométhyl-3,5,5-triméthylcyclohexane (diisocyanate d'isophorone), bis-(4-isocyanatocyclohexyl)méthane, 1,3- et 1,4-bis(isocyanatométhyl)-cyclohexane, bis-(4-isocyanato-3-méthyl-cyclohexyl)-méthane, α,α,α',α'-tétraméthyl-1,3- et 1,4-diisocyanate de xylène, 1-isocyanato-1-méthyl-4(3)-isocyanato-méthylcyclohexane et 2,4- et 2,6-diisocyanate d'hexahydrotoluène, diisocyanate de toluène, diisocyanate de diphénylméthane, diisocyanate de pentane - biosourcés, et des isomères ou des combinaisons de quelconques de ceux-ci.

7. Procédé de production d'une polyurée comprenant la mise en réaction d'un polyisocyanate avec l'ester polyaspartique à faible teneur en amine primaire (LPA) selon l'une quelconque des revendications 1 à 3.

8. Procédé selon la revendication 7, le polyisocyanate étant choisi dans le groupe constitué par diisocyanate de 1,4-tétraméthylène, diisocyanate de 1,6-hexaméthylène, diisocyanate de 2,2,4-triméthyl-1,6-hexaméthylène, diisocyanate de 1,12-dodécaméthylène, 1,3- et 1,4-diisocyanate de cyclohexane, 1-isocyanato-2-isocyanato-méthylcyclopentane, 1-isocyanato-3-isocyanatométhyl-3,5,5-triméthylcyclohexane (diisocyanate d'isophorone), bis-(4-isocyanatocyclohexyl)méthane, 1,3- et 1,4-bis(isocyanatométhyl)-cyclohexane, bis-(4-isocyanato-3-méthyl-cyclohexyl)-méthane, α,α,α',α'-tétraméthyl-1,3- et 1,4-diisocyanate de xylène, 1-isocyanato-1-méthyl-4(3)-isocyanato-méthylcyclohexane et 2,4- et 2,6-diisocyanate d'hexahydrotoluène, diisocyanate de toluène, diisocyanate de diphénylméthane, diisocyanate de pentane - biosourcés, et des isomères ou des combinaisons de quelconques de ceux-ci.

9. Procédé de production d'un ester polyaspartique à faible teneur en amine primaire (LPA) comprenant la mise en réaction d'une diamine aliphatique, préférablement d'une diamine cycloaliphatique, avec un excès d'un récepteur d'addition de Michael, en la présence d'un alcool en C₁-C₁₀, préférablement l'éthanol,
l'ester polyaspartique à faible teneur en amine primaire (LPA) ayant une teneur en monoaspartate inférieure à 10 %, préférablement inférieure à 5 %, particulièrement préférablement inférieure 3 %,
le récepteur d'addition de Michael étant choisi dans le groupe constitué par le malonate de diméthyle, le malonate de diéthyle, le malonate de dibutyle, le fumarate de diméthyle, le fumarate de diéthyle, le fumarate de dibutyle et des combinaisons correspondantes.

10. Procédé selon la revendication 9, la diamine cycloaliphatique étant choisie dans le groupe constitué par isophoronediamine (5-amino-(1-aminométhyl)-1,3,3-triméthylcyclohexane), 1,3-cyclohexanebis(méthylamine), 1,4-cyclohexanebis(méthylamine), 4,4'-diaminodicyclohexylméthane (PACM 20), bis(4-amino-3-méthylcyclohexyl)méthane, 3,3'-diméthyl-4,4'-diaminodicyclohexyl-méthane (DMDC), des isomères correspondants, des sels correspondants, des complexes correspondants, des adduits correspondants et de quelconques mélanges correspondants.

11. Procédé selon l'une quelconque des revendications 9 à 10, le récepteur d'addition de Michael étant compris en un excès d'au moins 50 %.

12. Revêtement, adhésif, agent d'étanchéité, film, composite, moulage ou peinture comprenant l'ester polyaspartique à faible teneur en amine primaire (LPA) préparé selon le procédé selon l'une quelconque des revendications 9 à 11.
